(19) Europäisches Patentamt
European Patent Office
Office européen des brevets

(11) Veröffentlichungsnummer : **0 438 377 A2**

# (12) EUROPÄISCHE PATENTANMELDUNG

(21) Anmeldenummer : **91810010.8**

(22) Anmeldetag : **09.01.91**

(51) Int. Cl.[5] : **C07D 409/12, A01N 43/54, A01N 43/60**

(30) Priorität : **18.01.90 CH 163/90**

(43) Veröffentlichungstag der Anmeldung : **24.07.91 Patentblatt 91/30**

(84) Benannte Vertragsstaaten : **AT BE CH DE DK ES FR GB GR IT LI LU NL**

(71) Anmelder : **CIBA-GEIGY AG**
**Klybeckstrasse 141**
**CH-4002 Basel (CH)**

(72) Erfinder : **Eckhardt, Wolfgang, Dr.**
**Breslauerstrasse 14**
**W-7850 Lörrach (DE)**
Erfinder : **Müller, Urs, Dr.**
**Drosselstrasse 6**
**CH-4142 Münchenstein (CH)**

## (54) Nematizide und fungizide Mittel.

(57) Es werden 2-(pyrimidyl oder Pyrazinyl)-imino-1,3-dithietan

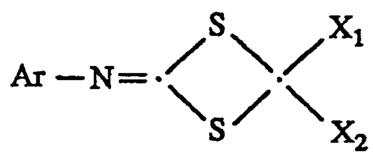 (I),

in welcher Ar die Reste

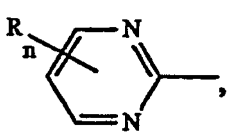, 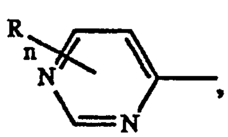, 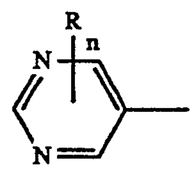, 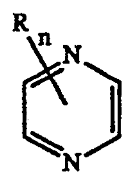,

R unsubstituiertes oder unabhängig voneinander durch Halogen, $C_1$-$C_3$Alkyl, $C_1$-$C_3$Alkoxy, $C_1$-$C_3$Alkylthio oder Cyano substituiertes $C_1$-$C_3$Alkyl, unsubstituiertes oder durch Halogen substituiertes $C_3$-$C_7$Alkenyl, unsubstituiertes oder durch Halogen substituiertes $C_3$-$C_7$Alkinyl, ferner $C_3$-$C_5$Cycloalkyl, $C_1$-$C_3$Alkoxy, $C_1$-$C_3$Alkylthio, Di-$C_1$-$C_3$alkylamino, Nitro, Halogen,
n die Zahlen 0-3,
$X_1$ und $X_2$ voneinander unabhängig Wasserstoff oder Halogen bedeuten, einschliesslich der mit Säuren der Formel $H^{\oplus}X^{\ominus}$ gebildeten Additionssalze, wobei das Anion $X^{\ominus}$ für $Cl^{\ominus}$, $Br^{\ominus}$, $J^{\ominus}$, $HSO_4^-$, $H_2PO_4^-$, $H_2PO_3^-$, $NO_3^-$, $CH_3COO^{\ominus}$, $CH_2ClCOO^-$, $CF_3COO^-$, $HOCH_2COO^-$, $CH_3CH(OH)COO^{\ominus}$, $HOOCCOO^{\ominus}$, $HOOCCH_2COO^{\ominus}$, $HOOCCH{=}CHCOO^{\ominus}$,

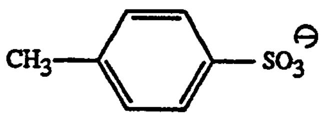 oder 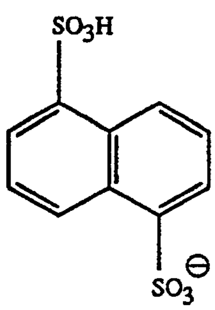 steht,

Verfahren zur Herstellung der Verbindungen der Formel I, sowie neue Zwischenprodukte des Herstellungsverfahrens beschrieben.

EP 0 438 377 A2

Die Verbindungen der Formel I besitzen nematizide und fungizide Eigenschaften. Es werden nematizide und fungizide Mittel, die als Wirkstoff mindestens einen Wirkstoff der Formel I enthalten, ferner Verfahren zur Verwendung der Wirkstoffe und der Mittel bei der Behämpfung von Nematoden und Pilzen beschrieben.

## NEMATIZIDE UND FUNGIZIDE MITTEL

Die vorliegende Erfindung betrifft neue 2-(Pyrimidyl oder Pyrazinyl)-imino-1,3-dithietane, deren Herstellung sowie nematizide Mittel, die als Wirkstoff mindestens eine dieser Verbindungen enthalten. Die Erfindung betrifft ferner neue Zwischenprodukte des Verfahrens zur Herstellung der Wirkstoffe, ihre Verwendung und Mittel zur Bekämpfung von Nematoden und Pilzen, insbesondere von pflanzenschädigenden Nematoden und Pilzen

Die Erfindung betrifft 2-(Pyrimidyl oder Pyrazinyl)-imino-1,3-dithietane der Formel I

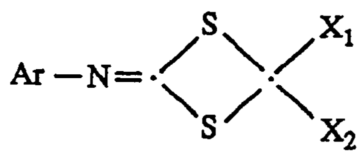

(I),

in welcher
Ar die Reste

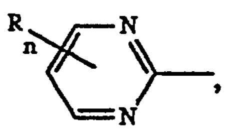 , 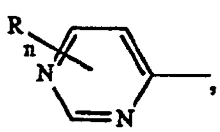 , 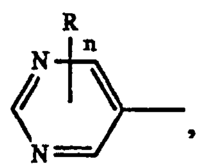 , 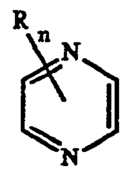 ,

R unsubstituiertes oder unabhängig voneinander durch Halogen, $C_1$-$C_3$Alkyl, $C_1$-$C_3$Alkoxy, $C_1$-$C_3$Alkylthio oder Cyano substituiertes $C_1$-$C_3$Alkyl, unsubstituiertes oder durch Halogen substituiertes $C_3$-$C_7$Alkenyl, unsubstituiertes oder durch Halogen substituiertes $C_3$-$C_7$Alkinyl, ferner $C_3$-$C_5$Cycloalkyl, $C_1$-$C_3$Alkoxy, $C_1$-$C_3$Alkylthio, Di-$C_1$-$C_3$alkylamino, Nitro, Halogen,
n die Zahlen 0-3,
$X_1$ und $X_2$ voneinander unabhängig Wasserstoff oder Halogen bedeuten, einschliesslich der mit Säuren der Formel $H^{\oplus}X^{\ominus}$ gebildeten Additionssalze, wobei das Anion $X^{\ominus}$ für $Cl^{\ominus}$, $Br^{\ominus}$, $J^{\ominus}$, $HSO_4^-$, $H_2PO_4^-$, $H_2PO_3^-$, $NO_3^-$, $CH_3COO^{\ominus}$, $CH_2ClCOO^-$, $CF_3COO^-$, $HOCH_2COO^-$, $CH_3CH(OH)COO^{\ominus}$, $HOOCCOO^{\ominus}$, $HOOCCH_2COO^{\ominus}$, $HOOCCH{=}CHCOO^{\ominus}$,

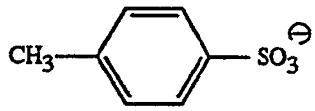 oder 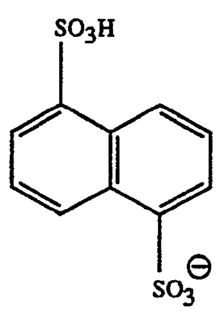 steht.

Unter Alkyl sind als selbständiger Rest sowie als Teil einer anderen Gruppe, wie Alkoxy, Alkylthio, Dialkylamino, die Gruppen Methyl, Ethyl sowie Normal- und und Isopropyl zu nennen. Halogensubstituiertes Alkyl steht für einen einfach bis perhalogenierten Alkylrest, wie z.B. $CHCl_2$, $CH_2F$, $CCl_3$, $CH_2Cl$ ; $CHFCH_2$, $CH_2CH_2Br$, $CF_2CF_3$, $C_2Cl_5$, $CH_2Br$, $CHBrCl$ usw. Für Alkylthio ist Ethylthio als Beispiel zu nennen. Alkenyl bedeutet z.B. Propenyl-(1), Allyl, Butenyl-(1), Butenyl-(2) oder Butenyl-(3) sowie Ketten mit mehreren Doppelbindungen. Alkinyl bedeutet z.B. Propinyl-(2), Butinyl-(1), Butinyl-(2), Pentinyl-(4) usw.. Halogen steht für Fluor, Chlor, Brom oder Jod, vorzugsweise für Chlor.

Beispiele salzbildender Säuren sind unter den anorganischen Säuren Halogenwasserstoffsäuren wie Chlorwasserstoff- säure, Bromwasserstoffsäure oder Jodwasserstoffsäure sowie ferner Schwefligesäure, Schwefelsäure, Phosphorsäure, phosphorige Säure, Salpetersäure und unter den organischen Säuren Essigsäure, Trifluoressigsäure, Monochloressigsäure, Glycolsäure, Bernsteinsäure, Oxalsäure, Maleinsäure,

Fumarsäure, p-Toluolsulfonsäure, und Naphthalin-1,5-disulfonsäure.

Es sind bereits nematizid wirksame Salze des 2-(2-Pyridylimino)-1,3-thietans aus der EP-A-310540 bekannt, welche jedoch in der Behauptung von schädlichen Nematoden die gestellten Ansprüche nicht im vollen Umfang befriedigen können.

Durch die Bereitstellung der erfindungsgemässen Verbindungen der Formel I ist es nun gelungen, einen wertvollen Beitrag zur Bekämpfung der beträchtliche landwirtschaftliche Schäden an Pflanzgut verursachenden Pflanzennematoden und Pilzen zu leisten. Auf diese Weise können Ernteeinbussen bei Kulturpflanzen wie z.B. Kartoffeln, Getreide, Rüben, Raps, Kohl, Tabak, Sojabohnen, Baumwolle, Mais, Reis und Gemüse sowie Schäden in Baumschulen und im Zierpflanzenbau nachhaltig eingedämmt werden. Die erfindungsgemässen Verbindungen zeichnen sich dabei insbesondere in der wirkungsvollen Bekämpfung von wurzelparasitären Bodennematoden wie z.B. solchen der Gattungen Heterodera und Globodera (zystenbildende Nematoden), Meloidogyne (Wurzelgallennematoden) sowie der Gattungen Radopholus, Pratylenchus, Tylenchulus, Longidorus, Trichodorus und Xiphinema aus. Ferner lassen sich mit den erfindungsgemässen Wirkstoffen die Nematodengattungen Ditylenchus (Stengelparasiten) Aphelenchoides (Blattnematoden) und Anguina (Blütennematoden) wirkungsvoll bekämpfen.

Mit den Wirkstoffen der Formel I lassen sich vorzugsweise besonders schädliche Nematodenarten der Gattung Meloidogyne, wie z.B. Meloidogyne incognita, sowie der Gattung Heterodera, wie z.B. Heterodera glycines (Sojabohnen-Zystennematode) und ferner der Gattung Globodera, wie z.B. Globodera rostochiensis (Kartoffelzystennematode) sowie Vertreter von wandernden Endoparasiten, wie z.B. Pratylenchus penetrans oder Radopholus similis und Vertreter von Ektoparasiten, wie z.B. Trichodorus spp. und Xiphinema spp. erfolgreich bekämpfen.

Ebenso lassen sich vorzugsweise mit den Wirkstoffen der Formel I besonders schädliche Pilze wie Ascomyceten, wie Erysiphe gramini, Fungi imperfecti, wie Botrytis cinerea und Cercospora beticola erfolgreich bekämpfen.

Zur Bekämpfung der Pflanzennematoden und Bodenpilze und zur Gesunderhaltung des Pflanzgutes können die neuen Wirkstoffe kurativ, präventiv oder systemisch eingesetzt werden. Dabei entfalten sie eine breit gefächerte Aktivität gegen die verschiedenen Nematoden- und Bodenpilzspecies und werden somit den Erfordernissen der Praxis gerecht. Die nematizide und fungizide Wirkungsweise der erfindungsgemässen Verbindungen wird durch eine geringe Phytotoxizität in vorteilhafter Weise begleitet, womit der allgemein wünschenswerten Verminderung der Umweltbelastung in besonderem Masse Rechnung getragen wird.

Eine bevorzugte Untergruppe sind Verbindungen der Formel I, bei welchen R Methyl, Methoxy, Methylthio, Chlor, n die Zahlen 0 bis 2 $X_1$ und $X_2$ voneinander unabhängig Wasserstoff oder Fluor bedeuten.

Von den obengenannten Verbindungen sind wegen ihrer nematiziden Wirkung jene insbesondere bevorzugt bei welchen R Methyl, Methoxy oder Methylthio, n die Zahlen 0 oder 1 und $X_1$ und $X_2$ Wasserstoff bedeuten.

Hervorzuheben sind von diesen Verbindungen jene der Formel Ia, Ib, Ic und Id

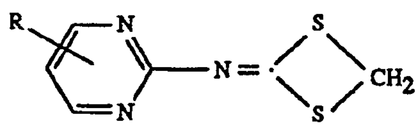

(Ia),

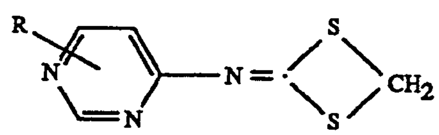

(Ib),

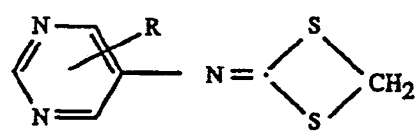

(Ic),

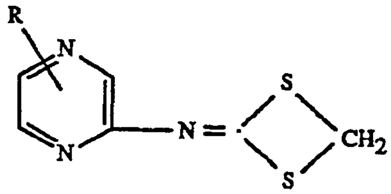

(Id).

Von den Verbindungen der Formel Ia ist das 2-(2-Pyrimidyl)-imino-1,3-dithietan hervorzuheben.

Von den Verbindungen der Formel Ib ist das 2-(4-Pyrimidyl)-imino- 1,3-dithietan zu nennen.

Aehnlich kann von den Verbindungen der Formel Ic das 2-(5-Pyrimidyl)-imino- 1,3-dithietan hervorgehoben werden.

Schliesslich ist von den Verbindungen der Formel Id das 2-(2-Pyrazinyl)-imino-1,3-dithietan von Bedeutung.

Verbindungen der Formel I werden erfindungsgemäss hergestellt, indem man ein Pyrimidyl- oder Pyrazinyl-amin der Formel II mit Schwefelkohlenstoff und mit einer Base B, mit oder ohne Lösungsmittel, bei -10 bis 50°C in ein Addukt der Formel III

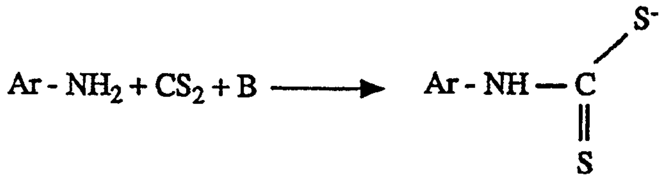

II III

,

letzteres mit einem Dibromethan-Derivat der Formel IV oder dem entsprechenden Dijod-Derivat und mit einer Base mit oder ohne Lösungsmittel bei -10 bis 100°C in das Reaktionsprodukt der Formel I

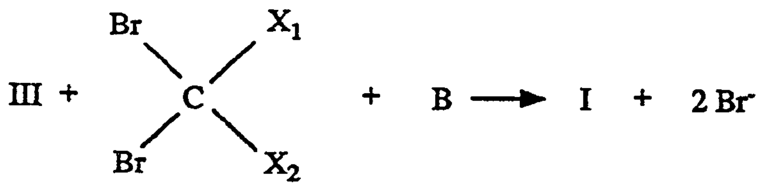

IV

überführt und dieses isoliert oder mit einer Säure der Formel $H^{\oplus}X^{\ominus}$ in das Additionssalz $I.H^{\oplus}X^{\ominus}$ verwandelt, wobei die Reste Ar, $X_1$, $X_2$ und $X^{\ominus}$ die bei der Definition der Formel I angegebenen Bedeutungen haben und B für ein Hydroxyd, ein Hydrid oder ein Carbonat eines Alkali- oder Erdalkalimetalls oder für ein tertiäres Ami steht. Dieses Verfahren ist auch Gegenstand der vorliegenden Erfindung.

Die Ausgangsprodukte der Formeln II und IV sind allgemein bekannt und können nach bekannten Verfahren hergestellt werden.

Nach einer bevorzugten Ausführungsform des erfindungsgemässen Verfahrens wird die Herstellung des Addukts der Formel III in einem Lösungsmittel bei 0 bis 40°C und die Umsetzung des letzteren mit dem Dibrommethan-Derivat der Formel IV in einem Lösungsmittel bei 0 bis 80°C durchgeführt.

Hervorzuheben ist diese Ausführungsform, wenn B ein Trialkylamin oder ein Hydrid, ein Hydroxyd oder ein Carbonat eines Alkali- oder Erdalkalimetalls bedeutet.

Für die Herstellung der erfindungsgemässen Wirksubstanzen geeignete Lösungs- oder Verdünnungsmittel sind z.B. Alkohole wie z.B. Methanol, Ethanol, Isopropanol oder Butanol ; N,N-dialkylierte Amide wie Dimethylformamid ; Dimethylsulfoxid ; Ether und etherartige Verbindungen wie Dialkylether (Diethylether, Diisopropylether, tert.-Butylmethylether usw.), Anisol, Dioxan, Tetrahydrofuran ; aliphatische und aromatische Kohlenwasserstoffe wie Benzol, Toluol, Petrolether, halogenierte Kohlenwasserstoffe wie Chlorbenzol, Methylenchlorid, Chloroform, Ethylenchlorid, Tetrachlorkohlenstoff, Tetrachlorethylen ; Nitrile wie Acetonitril, Propionitril ; Ketone wie Aceton, Diethylketon, Methylethylketon sowie Wasser und ganz allgemein Gemische solcher Lösungsmittel untereinander.

Als Basen kommen organische und anorganische Basen in Betracht ; z.B. vorzugsweise tertiäre Amine wie Trialkylamine (Trimethylamin, Triethylamin, Tripropylamin usw.), sowie Oxide, Hydroxide, Carbonate und Hydrogencarbonate von Alkali- und Erdalkalimetallen (z.B. CaO, BaO, NaOH, KOH, $Ca(OH)_2$, $KHCO_3$, $NaHCO_3$, $Ca(HCO_3)_2$, $K_2CO_3$, $Na_2CO_3$ usw.), ferner Acetate wie z.B. $CH_3COONa$ oder $CH_3COOK$. Darüber hinaus eignen sich als Basen auch Alkalialkoholate wie z.B. Natriumethylat, Natriumpropylat, Kalium-tert.-butylat oder Natriumethylat, sowie Alkalihydride wie beispielsweise Natriumhydrid

Die Erfindung betrifft auch Mittel, zur Bekämpfung von pflanzenschädigenden Nematoden sowie zur präventiven Verhütung des Nematodenbefalls von Pflanzgut, welche die Wirkstoffe der Formel I enthalten.

Darüber hinaus schliesst die vorliegende Erfindung zusätzlich die Herstellung nematizider und fungizider Mittel ein, die gekennzeichnet ist durch das innige Vermischen von Aktivsubstanzen der Formel I mit einer oder mehreren hierin beschriebenen Trägerstoffen und Hilfsingredienzen. Eingeschlossen ist auch ein Verfahren zur Behandlung von Pflanzen, das durch die Applikation der Verbindungen der Formel I oder der neuen Mittel charakterisiert ist.

Ein bezvorzugtes Verfahren für den Einsatz eines Wirkstoffs der Formel I bzw. eines nematiziden Mittels, das mindestens einen dieser Wirkstoffe enthält, ist das Einbringen in den Erdboden. Dabei wird der Standort der Pflanzen mit einer flüssigen oder festen Zubereitung behandelt

Die Verbindungen der Formel I können aber auch auf Samenkörner aufgebracht werden (Beizung/Coating), indem man die Körner entweder in einer flüssigen Zubereitung des Wirkstoffs tränkt oder sie mit einer festen Zubereitung beschichtet Darüber hinaus sind in besonderen Fällen weitere Applikationsarten möglich, so z.B. die gezielte Behandlung der Pflanzenstengel, Knospen oder Blätter.

Wirkstoffe der Formel I werden üblicherweise in Form von formulierten Zusammensetzungen verwendet und können gleichzeitig oder nacheinander mit weiteren Wirkstoffen auf die zu behandelnde Fläche oder Pflanze gegeben werden. Diese weiteren Wirkstoffe können auch andere in der Landwirtschaft angewendete Mittel umfassen, die in ihrer Nutzanwendung der Produktionssteigerung durch Förderung des Nutzpflanzenwachstums dienen, wie Düngemittel, Herbizide, Insektizide, Fungizide, Molluskizide u.a., oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Die Verbindungen der Formel I werden in unveränderter Form oder vorzugs- weise zusammen mit den in der Formulierungstechnik üblichen Hilfsmitteln eingesetzt. Sie werden z.B. zu Emulsionskonzentraten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Verstäuben, Verstreuen oder Giessen werden gleich wie die Art der Mittel den angestrebten Zielen und den gegebenen Verhältnissen entsprechend gewählt. Günstige Aufwandmengen liegen im allgemeinen bei 300 g bis 6 kg Aktivsubstanz (AS) je ha ; bevorzugt 0,3 bis 2 kg AS/ha.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenfalls mit oberflächenaktiven Substanzen (Tensiden).

Als Lösungsmittel kommen in Frage : Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl ; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur, wie insbesondere Dolomit oder zerkleinerte Pflanzenrückstände, verwendet werden.

Als oberflächenaktive Substanzen kommen je nach Art des zu formulierenden Wirkstoffes der Formel I nichtionogene, kation- und/oder anionaktive Tenside mit guten Emulgier-, Dispergier- und Netzeigenschaften

in Betracht Unter Tensiden sind auch Tensidgemische zu verstehen.

Geeignete anionische Tenside können sowohl sogenannte wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnussoder Talgöl gewonnen werden können. Ferner sind auch die Fettsäuremethyllaurinsalze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylsulfonate.

Die Fettalkoholsulfonate oder -sulfate liegen in der Regel als Alkali-, Erdalkali- oder gegebenenfalls substituierte Ammoniumsalze vor und weisen einen Alkalirest mit 8 bis 22 C-Atomen auf, wobei Alkyl auch den Alkylteil von Acylresten einschliesst, z.B. Das Naoder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol- Ethylenoxyd-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die neuen Formulierungstechnik gebräuchlichen Tenside sind u.a. in folgenden Publikationen beschrieben:

"Mc Cutcheon's Detergents and Emulsifiers Annual" MC Publishing Corp., Ridgewood, New Jersey, 1979; Dr. Helmut Stache "Tensid Taschenbuch", Carl Hanser Verlag München/Wien.

Die agrochemischen Zubereitungen enthalten in der Regel 0,1 bis 99 Gew.-%, insbesondere 0,1 bis 95 Gew.-%, Wirkstoff der Formel I", 99,9 bis 1 Gew.-%, insbesondere 99,8 bis 5 Gew.-%, eines festen oder flüssigen Zusatzstoffes und 0 bis 25 Gew.-%, insbesondere 0,1 bis 25 Gew.-%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Die nachfolgenden Beispiele dienen zur näheren Erläuterung der Erfindung, ohne dieselbe einzuschränken.

1. Herstellungsbeispiele :

2-(5-Pyrimidylimino)-1,3-dithietan (Verbindung 1.1)

Zu einer vorbereiteten Lösung von 20 g (0,211 Mol) 5-Aminopyrimidin und 12,7 ml (0,211 Mol) Schwefelkohlenstoff in 50 ml Ethylalkohol werden 23,4 g (0,232 Mol) Triethylamin zugetropft. Das Reaktionsgemisch wird anschliessend während 15 Stunden bei 70°C gerührt und die entstandene Suspension wird abfiltriert. Nach Trocknen erhält man 10 g Schwefelkohlenstoffaddukt. Das Filtrat wird eingedampft und man erhält weitere 27 g des Zwischenproduktes als Rückstand.

Zu 10 g (0,037 Mol) des Zwischenproduktes in 40 ml Dimethylformamid werden eine Mischung von 41,9 g (0,241 Mol) Dibrommethan und 3,7 g (0,037 Mol) Triethylamin in 20 ml Dimethylformamid bei 25°C unter Rühren zugetropft. Das Reaktionsgemisch wird bei 25°C 20 Stunden gerührt, dann auf Eiswasser gegossen und mit Diethylether extrahiert. Die vereinigten Diethyletherextrakte werden 3 mal mit je 100 ml Wasser extrahiert, mit Natriumsulfat getrocknet, filtriert und eingedampft. Das Rohprodukt wird über eine Kieselgel-Flash-Säule mit Essigsäureethylester/Hexan (2/1) gereinigt. Man erhält so 2,7 g (40 % d.Th.) Produkt vom Smp. 97-99°C.

2-(5-Pyrimidylimino)-1,3-dithietanhydrochlorid (Verbindung 1.2)

In eine Lösung von 0,5 g (2,732 mmol) 2-(5-Pyrimidylimino)-1,3-dithietan in 50 ml Diethylether wird während einer halben Stunde bei 0-10°C trockenes HCl-Gas eingeleitet. Das ausfallende Produkt wird abfiltriert, mit Diethylether gewaschen und getrocknet. Man erhält so 0,5 g (83,4 % d.Th.) Produkt vom Smp. (Zers.) ab 150°C.

Die in den folgenden Tabellen 1-4 aufgeführten Verbindungen können in Analogie zu den obigen Beispielen hergestellt werden.

Tabelle 1 : 2-(5-Pyrimidyl)-imino-1,3-dithietane

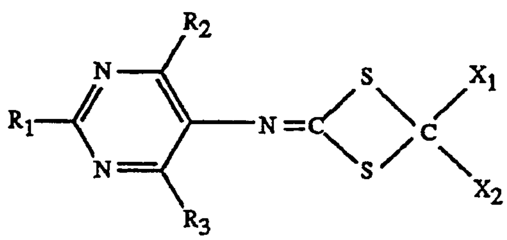

. HX (Ia).HX

| Verb. Nr. | $R_1$ | $R_2$ | $R_3$ | $X_1$ | $X_2$ | HX | Physik. Daten |
|---|---|---|---|---|---|---|---|
| 1.1 | H | H | H | H | H | - | Smp. 97-99°C |
| 1.2 | H | H | H | H | H | HCl | Smp. >150°C (Zers.) |
| 1.3 | H | H | H | H | H | $H_2SO_4$ | Smp. >110°C (Zers.) |
| 1.4 | H | H | H | H | H | HBR | |
| 1.5 | H | H | H | H | H | HJ | |
| 1.6 | H | H | H | H | H | $HNO_3$ | |
| 1.7 | H | H | H | H | H | $H_2SO_3$ | |
| 1.8 | H | H | H | H | H | $H_3PO_4$ | |
| 1.9 | H | H | H | H | H | $CH_3COOH$ | |
| 1.10 | H | H | H | H | H | $(COOH)_2$ | |
| 1.11 | H | H | H | H | H | $ClCH_2COOH$ | |
| 1.12 | H | H | H | H | H | $HOCH_2COOH$ | |
| 1.13 | H | H | H | H | H | $CH_2(COOH)_2$ | |
| 1.14 | H | H | H | H | H | CH-COOH<br>‖<br>CH-COOH | |
| 1.15 | H | H | H | H | H | 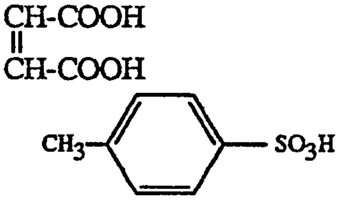 | |
| 1.16 | H | H | H | H | H | 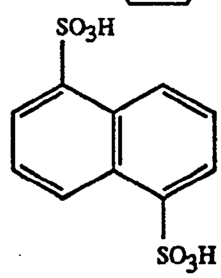 | |
| 1.17 | $CH_3$- | H | H | H | H | - | |
| 1.18 | $CH_3$ | H | H | H | H | HCl | |
| 1.19 | $CH_3$ | H | H | H | H | $H_2SO_4$ | |
| 1.20 | H | H | H | F | F | - | |
| 1.21 | H | H | H | Cl | Cl | - | |
| 1.22 | $CH_3$ | H | H | F | F | - | |

Tabelle 2 : 2-(4-Pyrimidyl)-imino-1,3-dithietane

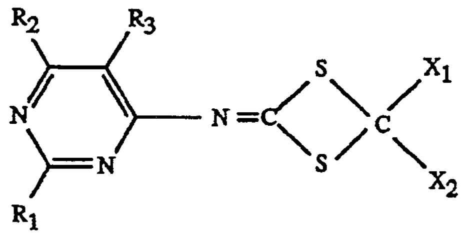

. HX (Ib).HX

| Verb. Nr. | $R_1$ | $R_2$ | $R_3$ | $X_1$ | $X_2$ | HX | Physik. Daten |
|---|---|---|---|---|---|---|---|
| 2.1 | H | H | H | H | H | - | Smp. 153-155°C |
| 2.2 | H | H | H | H | H | HCl | |
| 2.3 | H | H | H | H | H | $H_2SO_4$ | |
| 2.4 | H | H | H | H | H | $CH_3COOH$ | |
| 2.5 | H | H | H | H | H | 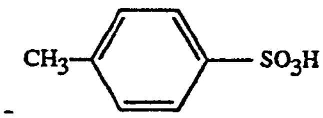 | |
| 2.6 | $CH_3$ | $CH_3$ | H | H | H | - | |
| 2.7 | $CH_3$ | $CH_3$ | H | H | H | HCl | |
| 2.8 | $CH_3$ | $CH_3$ | H | H | H | $H_2SO_4$ | |
| 2.9 | $CH_3S$ | Cl | H | H | H | - | |
| 2.10 | $CH_3S$ | Cl | H | H | H | HCl | |
| 2.11 | $CH_3S$ | Cl | H | H | H | $H_2SO_4$ | |
| 2.12 | H | H | H | F | F | - | |
| 2.13 | H | H | H | Cl | Cl | - | |
| 2.14 | $CH_3S$ | H | Cl | F | F | - | |
| 2.15 | $CH_3O$ | H | $CH_3O$ | F | F | - | |

Tabelle 3 : 2-(2-Pyrimidyl)-imino-1,3-dithietane

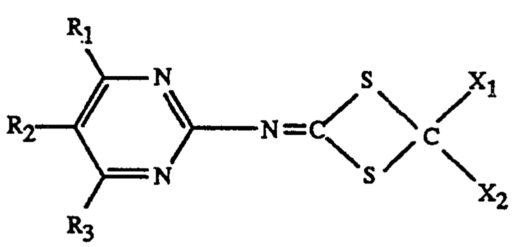

. HX (Ic).HX

| Verb. Nr. | $R_1$ | $R_2$ | $R_3$ | $X_1$ | $X_2$ | HX | Physik. Daten |
|---|---|---|---|---|---|---|---|
| 3.1 | H | H | H | H | H | - | Smp.: 173-175°C |
| 3.2 | H | H | H | H | H | HCl | |
| 3.3 | H | H | H | H | H | $H_2SO_4$ | |
| 3.4 | H | H | H | H | H | $CH_3COOH$ | |
| 3.5 | H | H | H | H | H | $HNO_3$ | |
| 3.6 | H | H | H | H | H | 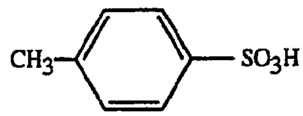 | |
| 3.7 | H | Br | H | H | H | - | |
| 3.8 | H | Br | H | H | H | HCl | |
| 3.9 | H | Br | H | H | H | $H_2SO_4$ | |
| 3.10 | H | $CH_3$ | H | H | H | - | Smp. 102-103°C |
| 3.11 | H | $CH_3$ | H | H | H | HCl | |
| 3.12 | $CH_3$ | H | $CH_3$ | H | H | - | |
| 3.13 | $CH_3$ | H | $CH_3$ | H | H | HCl | |
| 3.14 | $CH_3$ | H | $CH_3$ | H | H | $H_2SO_4$ | |

Tabelle 3 (Fortsetzung)

| Verb. Nr. | $R_1$ | $R_2$ | $R_3$ | $X_1$ | $X_2$ | HX | Physik. Daten |
|---|---|---|---|---|---|---|---|
| 3.15 | $CH_3$ | H | 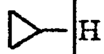 | H | H | - | |
| 3.16 | $CH_3$ | H | 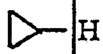 | H | H | HCl | |
| 3.17 | $CH_3$ | H | 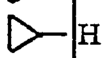 | H | H | $H_2SO_4$ | |
| 3.18 | $CH_3$ | H | H | H | H | - | |
| 3.19 | $CH_3$ | H | H | H | H | HCl | |
| 3.20 | $CH_3$ | H | H | H | H | $H_2SO_4$ | |
| 3.21 | H | $NO_2$ | H | H | H | - | |
| 3.22 | H | $NO_2$ | H | H | H | HCl | |
| 3.23 | H | $NO_2$ | H | H | H | $H_2SO_4$ | |
| 3.24 | H | H | H | F | F | - | |
| 3.25 | H | H | H | Cl | Cl | - | |
| 3.26 | H | Br | H | F | F | - | |
| 3.27 | H | $CH_3$ | H | F | F | - | |
| 3.28 | $CH_3$ | H | $CH_3$ | F | F | - | |
| 3.29 | $CH_3$ | H | 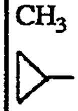 | F | F | - | |
| 3.30 | H | $NO_2$ | H | F | F | - | |
| 3.31 | Cl | H | $CH_3$ | H | H | - | |
| 3.32 | Cl | H | $CH_3$ | H | H | HCl | |
| 3.33 | Cl | H | $CH_3$ | H | H | $H_2SO_4$ | |
| 3.34 | Cl | H | $CH_3$ | F | F | - | |
| 3.35 | Cl | H | Cl | H | H | - | |
| 3.36 | $CH_3$ | H | $OCH_3$ | H | H | - | |
| 3.37 | $CH_3$ | H | $OCH_3$ | H | H | HCl | |
| 3.38 | $CH_3$ | H | $OCH_3$ | H | H | $H_2SO_4$ | |

Tabelle 4 : 2-(2-Pyrazinyl)-imino-1,3-dithietane

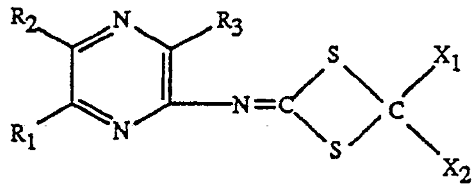 . HX (Id).HX

| Verb. Nr. | $R_1$ | $R_2$ | $R_3$ | $X_1$ | $X_2$ | HX | Physik. Daten |
|---|---|---|---|---|---|---|---|
| 4.1 | H | H | H | H | H | - | Smp. 154-156°C |
| 4.2 | H | H | H | H | H | HCl | Smp. 162-165°C |
| 4.3 | H | H | H | H | H | $H_2SO_4$ | Smp. 158-159°C |
| 4.4 | H | H | H | H | H | 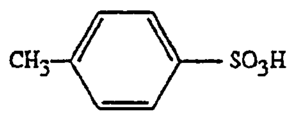 | |
| 4.5 | H | H | H | F | F | - | |
| 4.6 | H | H | H | F | F | HCl | |
| 4.7 | H | H | H | F | F | $H_2SO_4$ | |
| 4.8 | $CH_3$ | H | H | H | H | - | |
| 4.9 | $CH_3$ | $CH_3$ | H | H | H | - | |
| 4.10 | $CH_3$ | $CH_3$ | H | F | F | - | |
| 4.11 | H | H | $CH_3$ | H | H | - | |

2. Formulierungsbeispiele für flüssige Wirkstoffe der Formel I (%=Gewichtsprozent)

| 2.1 Emulsions-Konzentrate | a) | b) | c) |
|---|---|---|---|
| Wirkstoff aus den Tabellen 1-4 | 25 % | 40 % | 50 % |
| Ca-Dodecylbenzolsulfonat | 5 % | 8 % | 6 % |
| Ricinusöl-polyethylenglykolether (36 Mol Ethylenoxid) | 5 % | - | - |
| Tributylphenoyl-polyethylenglykol-ether (30 Mol Ethylenoxid) | - | - | 12 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2.2 Lösungen | a) | b) | c) | d) |
| --- | --- | --- | --- | --- |
| Wirkstoff aus den Tabellen 1-4 | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190°C) | - | - | 94 % | - |

(MG = Molekulargewicht)

Die Lösungen sind zur Anwendung in Form kleinster Tropfen geeignet.

| 2.3 Granulate | a) | b) |
| --- | --- | --- |
| Wirkstoff aus den Tabellen 1-4 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Wakuum abgedampft.

| 2.4 Stäubemittel | a) | b) |
| --- | --- | --- |
| Wirkstoff aus den Tabellen 1-4 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Formulierungsbeispiele für feste Wirkstoffe der Formel I (% = Gewichtsprozent)

| 2.5 Spritzpulver | a) | b) | c) |
| --- | --- | --- | --- |
| Wirkstoff aus den Tabellen 1-4 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 2.6 Emulsions-Konzentrat | |
|---|---|
| Wirkstoff aus den Tabellen 1-4 | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 2.7 Stäubemittel | a) | b) |
|---|---|---|
| Wirkstoff aus den Tabellen 1-4 | 5 % | 8 % |
| Talkum | 95 % | - |
| Kaolin | - | 92 % |

Man erhält anwendungsfertige Stäubemittel, indem der Wirkstoff mit den Trägern vermischt und auf einer geeigneten Mühle vermahlen wird.

| 2.8 Extruder Granulat | |
|---|---|
| Wirkstoff aus den Tabellen 1-4 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| 2.9 Umhüllungs-Granulat | |
|---|---|
| Wirkstoff aus den Tabellen 1-4 | 3 % |
| Polyethylenglykol (MG 200) | 3 % |
| Kaolin | 94 % |

(MG = Molekulargewicht)

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

2.10 Suspensions-Konzentrat

| | | |
|---|---|---|
| Wirkstoff aus den Tabellen 1-4 | 40 | % |
| Ethylenglykol | 10 | % |
| Nonylphenolpolyethylenglykolether (15 Mol Ethylenoxid) | 6 | % |
| N-Ligninsulfonat | 10 | % |
| Carboxymethylcellulose | 1 | % |
| 37%ige wässrige Formaldehyd-Lösung | 0,2 | % |
| Silikonöl in Form einer 75%igen wässrigen Emulsion | 0,8 | % |
| Wasser | 32 | % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

2. Biologische Beispiele

2.1 Wirkung gegen Meloidogyne incognita auf Tomaten

Eier von Meloidogyne incognita werden in Sand eingemischt. Dieses Gemisch wird dann in 200 ml fassende Tontöpfe eingebracht (5000 Eier pro Topf). Am gleichen Tag wird pro Topf eine 3 Wochen alte Tomatenpflanze gepflanzt und der formulierte Wirkstoff mittels Drenchapplikation in die Töpfe hineingegeben (0,0006 % Aktivsubstanz bezogen auf das Bodenvolumen). Die eingetopften Pflanzen werden nun bei einer Temperatur von 26 ± 1°C und einer relativen Luftfeuchtigkeit von 60 % in einem Gewächshaus aufgestellt. Nach Ablauf von 4 Wochen erfolgt eine Evaluierung durch Untersuchung der Pflanzen auf Wurzelgallbildung nach dem sogenannten Wurzelgallen-Index ("Root-Knot Index").

Die Verbindungen aus den Tabellen 1-4 zeigen gegen Meloidogyne incognita durch Reduktion der Wurzelgallbildung eine Wirksamkeit. Unbehandelte jedoch infizierte Kontrollpflanzen weisen dagegen starke Wurzelgallbildung auf (= 100 %). Eine gute Wirkung zeigen z.B. die Verbindungen Nr. 1.1, 1.2, 1.3, 3.1 und 4.1 mit weniger als 20 % Restbefall, die Verbindungen hemmen im obigen Versuch die Wurzelgallbildung sogar fast vollständig (0-10 % Restbefall).

2.2 Wirkung gegen Heterodera glycines auf Sojabohnen

Sandige Erde wird mit Eiern des Sojabohnenzystennematoden H. *glycines* infestiert, etwa 6000 Eier pro Topf. Anschliessend werden die zu prüfenden Substanzen in den entsprechenden Konzentrationen eingemischt. Die behandelte und infestierte Erde wird dann in 1c Töpfe (180 ccm) gefüllt und jeweils drei Sojabohen (cv. Maple Arrow) eingesät. Jede Behandlung wird dreimal wiederholt. Die Töpfe werden bei ca 27°C für vier bis fünf Wochen im Gewächshaus inkubiert. Die Pflanzen werden dann vorsichtig den Töpfen entnommen, die Wurzeln gewaschen und die Anzahl der Zysten bestimmt. Die Bonitur erfolgt entsprechend dem Wirkungsschema 1-9 (1 = volle Aktivität, 9 = keine Aktivität).

Die Verbindungen aus den Tabllen 1-4 zeigen gegen Heterodera glycines eine gute Wirkung, was sich in der fast vollständigen Reduktion der Zystenbildung zeigt

## Patentansprüche

1. 2-(Pyrimidyl oder Pyrazinyl)-imino-1,3-dithietane der Formel I

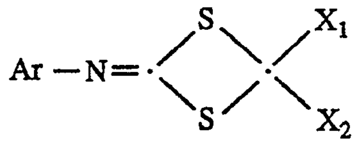 (I),

dadurch gekennzeichnet, dass
Ar die Reste

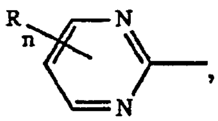, 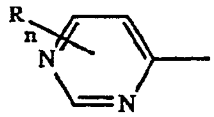, 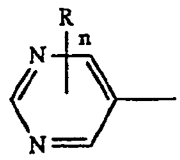, 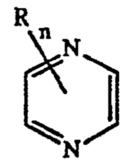,

R unsubstituiertes oder unabhängig voneinander durch Halogen, $C_1$-$C_3$Alkyl, $C_1$-$C_3$Alkoxy, $C_1$-$C_3$Alkylthio oder Cyano substituiertes $C_1$-$C_3$Alkyl, unsubstituiertes oder durch Halogen substituiertes $C_3$-$C_7$Alkenyl, unsubstituiertes oder durch Halogen substituiertes $C_3$-$C_7$Alkinyl, ferner $C_3$-$C_5$Cycloalkyl, $C_1$-$C_3$Alkoxy, $C_1$-$C_3$Alkylthio, Di-$C_1$-$C_3$alkylamino, Nitro, Halogen,
n die Zahlen 0-3,
$X_1$ und $X_2$ voneinander unabhängig Wasserstoff oder Halogen bedeuten, einschliesslich der mit Säuren der Formel $H^{\oplus}X^{\ominus}$ gebildeten Additionssalze, wobei das Anion $X^{\ominus}$ für $Cl^{\ominus}$, $Br^{\ominus}$, $J^{\ominus}$, $HSO_4^-$, $H_2PO_4^-$, $H_2PO_3^-$, $NO_3^-$, $CH_3COO^{\ominus}$ , $CH_2ClCOO^-$, $CF_3COO^-$, $HOCH_2COO^-$, $CH_3CH(OH)COO^{\ominus}$, $HOOCCOO^{\ominus}$, $HOOCCH_2COO^{\ominus}$ $HOOCCH{=}CHCOO^{\ominus}$,

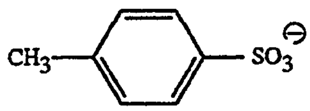 oder 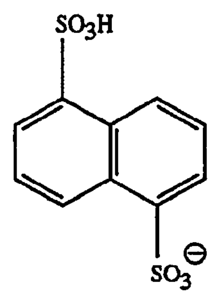 steht.

2. Verbindungen gemäss Anspruch 1, dadurch gekennzeichnet, dass R Methyl, Methoxy, Methylthio, Chlor, n die Zahlen 0 bis 2 und $X_1$ und $X_2$ voneinander unabhängig Wasserstoff oder Fluor bedeuten.

3. Verbindungen gemäss Anspruch 2, dadurch gekennzeichnet, dass R Methyl, Methoxy oder Methylthio, n die Zahlen 0 oder 1 und $X_1$ und $X_2$ Wasserstoff bedeuten.

4. Verbindung der Formel Ia

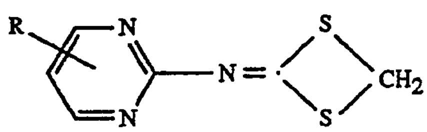 (Ia),

gemäss Anspruch 3.

5. Das 2-(2-Pyrimidyl)-imino-1,3-dithietan gemäss Anspruch 4.

6. Verbindung der Formel Ib

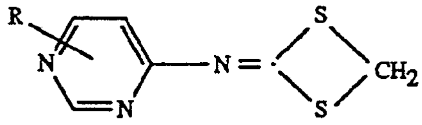

(Ib),

gemäss Anspruch 3.

7. Das 2-(4-Pyrimidyl)-imino-1,3-dithietan gemäss Anspruch 6.

8. Verbindung der Formel Ic

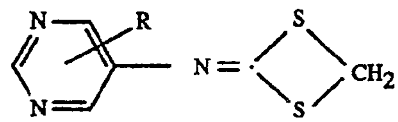

(Ic),

9. Das 2-(5-Pyrimidyl)-imino-1,3-dithietan gemäss Anspruch 8.

10. Verbindung der Formel Id

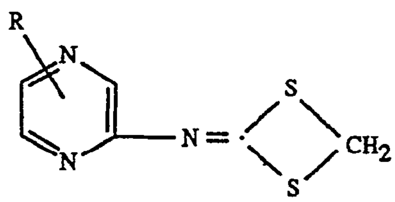

(Id),

gemäss Anspruch 3.

11. Das 2-(2-Pyrazinyl)-imino-1,3-dithietan gemäss Anspruch 10.

12. Verfahren zur Herstellung von 2-(Pyrimidyl oder Pyrazinyl)-imino-1,3-dithietanen der Formel I, Anspruch 1, dadurch gekennzeichnet, dass man ein Pyrimidyl- oder Pyrazinyl-amin der Formel II mit Schwefelkohlenstoff und mit einer Base B, mit oder ohne Lösungsmittel, bei -10 bis 50°C in ein Addukt der Formel III

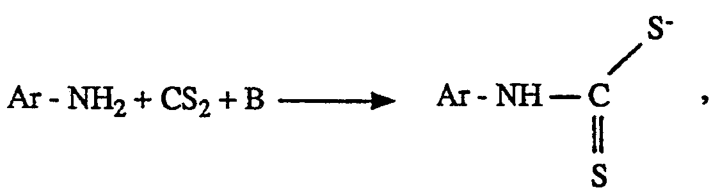

II III

letzteres mit einem Dibromethan-Derivat der Formel IV oder dem entsprechenden Dijod-Derivat und mit einer Base mit oder ohne Lösungsmittel bei -10 bis 100°C in das Reaktionsprodukt der Formel I

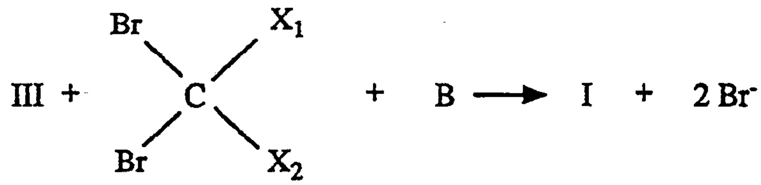


IV

überführt und dieses isoliert oder mit einer Säure der Formel $H^{\oplus}X^{\ominus}$ in das Additionssalz $I.H^{\oplus}X^{\ominus}$ verwandelt, wobei die Reste Ar, $X_1$, $X_2$ und $X^{\ominus}$ die im Anspruch 1 angegebenen Bedeutungen haben und B für ein Hydroxyd, ein Hydrid oder ein Carbonat eines Alkalioder Erdalkalimetalls oder für ein tertiäres Amin steht.

13. Verfahren gemäss Anspruch 12, dadurch gekennzeichnet, dass die Herstellung des Addukts der Formel III in einem Lösungsmittel bei 0 bis 40°C und die Umsetzung des letzteren mit dem Dibrommethan-Derivat der Formel IV in einem Lösungsmittel bei 0 bis 80°C durchgeführt wird.

14. Verfahren gemäss Anspruch 13, dadurch gekennzeichnet, dass B ein Trialkylamin oder ein Hydrid, ein Hydroxyd oder ein Carbonat eines Alkali- oder Erdalkalimetalls bedeutet.

15. Schädlingsbekämpfungsmittel zur Bekämpfung oder Verhütung des Befalls von Pflanzen durch Nematoden und Pilze, dadurch gekennzeichnet, dass es als aktive Komponente mindestens eine Verbindung der Formel I, gemäss Anspruch 1 enthält.

16. Verfahren zur Herstellung eines agrochemischen Mittels, dadurch gekennzeichnet, dass mindestens eine Verbindung der Formel I gemäss Anspruch 1 mit geeigneten festen oder flüssigen Zusatzstoffen und Tensiden innig vermischt.

17. Verfahren zur Bekämpfung oder Verhütung eines Befalls von Kultur- pflanzen durch Nematoden und Pilze, dadurch gekennzeichnet, dass man eine Verbindung der Formel I gemäss einem der Ansprüche 1 bis 11 auf die Pflanze oder deren Standort appliziert.

18. Verwendung von Verbindungen der Formel I gemäss Anspruch 1 zur Bekämpfung und/oder zur präventiven Verhütung eines Befalls von Pflanzen durch Nematoden und Pilze.

**Ansprüche Für folgenden Vertragsstaat : ES**

1. Verfahren zur Herstellung von 2-(Pyrimidyl oder Pyrazinyl)-imino-1,3-dithietanen der Formel I

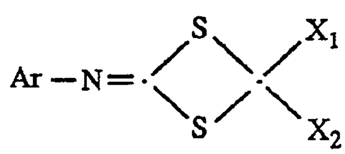

(I),

dadurch gekennzeichnet, dass
Ar die Reste

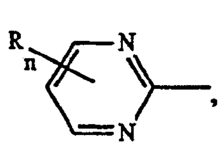
,
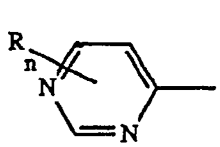
,
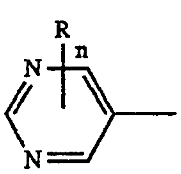
,
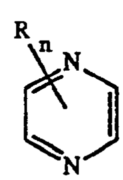
,

R unsubstituiertes oder unabhängig voneinander durch Halogen, $C_1$-$C_3$Alkyl, $C_1$-$C_3$Alkoxy, $C_1$-$C_3$Alkylthio oder Cyano substituiertes $C_1$-$C_3$Alkyl, unsubstituiertes oder durch Halogen substituiertes $C_3$-$C_7$Alkenyl, unsubstituiertes oder durch Halogen substituiertes $C_3$-$C_7$Alkinyl, ferner $C_3$-$C_5$Cycloalkyl, $C_1$-$C_3$Alkoxy, $C_1$-$C_3$Alkylthio, Di-$C_1$-$C_3$alkylamino, Nitro, Halogen,

n die Zahlen 0-3,

$X_1$ und $X_2$ voneinander unabhängig Wasserstoff oder Halogen bedeuten, einschliesslich der mit Säuren der Formel $H^{\oplus}X^{\ominus}$ gebildeten Additionssalze, wobei das Anion $X^{\ominus}$ für $CL^{\ominus}$, $Br^{\ominus}$, $J^{\ominus}$, $HSO_4^-$, $H_2PO_4^-$, $H_2PO_3^-$, $NO_3^-$, $CH_3COO^{\ominus}$, $CH_2ClCOO^-$, $CF_3COO^-$, $HOCH_2COO^-$, $CH_3CH(OH)COO^{\ominus}$, $HOOCCOO^{\ominus}$, $HOOCCH_2COO^{\ominus}$, $HOOCCH{=}CHCOO^{\ominus}$,

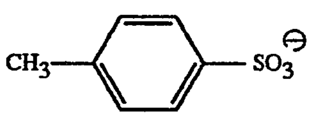 oder 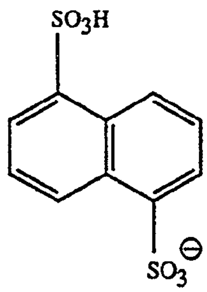 steht, dadurch gekennzeichnet, dass man

ein Pyrimidyl- oder Pyrazinyl-amin der Formel II mit Schwefelkohlenstoff und mit einer Base B, mit oder ohne Lösungsmittel, bei -10 bis 50°C in ein Addukt der Formel III

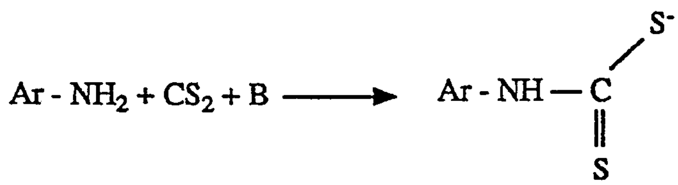 ,

II III

letzteres mit einem Dibromethan-Derivat der Formel IV oder dem entsprechenden Dijod-Derivat und mit einer Base mit oder ohne Lösungsmittel bei -10 bis 100°C in das Reaktionsprodukt der Formel I

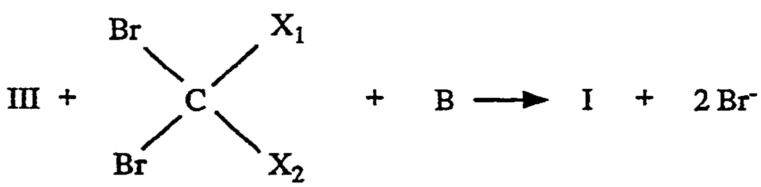

IV

überführt und dieses isoliert oder mit einer Säure der Formel $H^{\oplus}X^{\ominus}$ in das Additionssalz $I.H^{\oplus}X^{\ominus}$ verwandelt, wobei die Reste Ar, $X_1$, $X_2$ und $X^{\ominus}$ die im Anspruch 1 angegebenen Bedeutungen haben und B für ein Hydroxyd, ein Hydrid oder ein Carbonat eines Alkalioder Erdalkalimetalls oder für ein tertiäres Amin steht.

2. Verfahren gemäss Anspruch 1, dadurch gekennzeichnet, dass die Herstellung des Addukts der Formel III in einem Lösungsmittel bei 0 bis 40°C und die Umsetzung des letzteren mit dem Dibrommethan-Derivat der Formel IV in einem Lösungsmittel bei 0 bis 80°C durchgeführt wird.

3. Verfahren gemäss Anspruch 2, dadurch gekennzeichnet, dass B ein Trialkylamin oder ein Hydrid, ein Hydroxyd oder ein Carbonat eines Alkali- oder Erdalkalimetalls bedeutet.

4. Verfahren gemäss Anspruch 1, dadurch gekennzeichnet, dass R Methyl, Methoxy, Methylthio, Chlor, n die Zahlen 0 bis 2 und $X_1$ und $X_2$ voneinander unabhängig Wasserstoff oder Fluor bedeuten.

5. Verfahren gemäss Anspruch 4, dadurch gekennzeichnet, dass R Methyl, Methoxy oder Methylthio, n die Zahlen 0 oder 1 und $X_1$ und $X_2$ Wasserstoff bedeuten.

6. Verfahren zur Herstellung von Verbindungen der Formel Ia

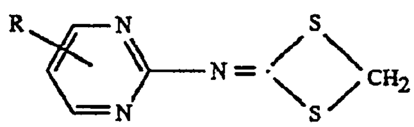

(Ia),

gemäss Anspruch 5.

7. Verfahren zur Herstellung von 2-(2-Pyrimidyl)-imino-1,3-dithietan gemäss Anspruch 6.

8. Verfahren zur Herstellung von Verbindungen der Formel Ib

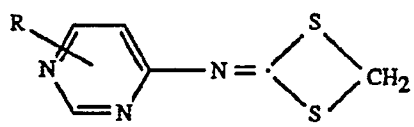

(Ib),

gemäss Anspruch 5.

9. Verfahren zur Herstellung von 2-(4-Pyrimidyl)-imino-1,3-dithietan gemäss Anspruch 8.

10. Verfahren zur Herstellung von Verbindungen der Formel Ic

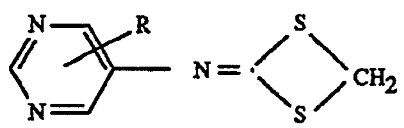

(Ic),

gemäss Anspruch 1.

11. Verfahren zur Herstellung von 2-(5-Pyrimidyl)-imino-1,3-dithietan gemäss Anspruch 10.

12. Verfahren zur Herstellung von Verbindungen der Formel Id

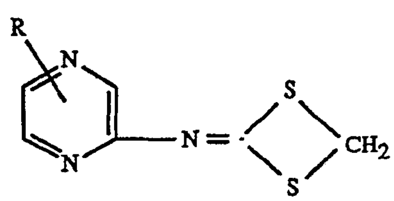

(Id),

gemäss Anspruch 5.

13. Verfahren zur Herstellung von 2-(2-Pyrazinyl)-imino-1,3-dithietan gemäss Anspruch 12.